# FASCICULE DE BREVET EUROPEEN

(11) **EP 2 735 297 B1**
(45) Date de publication et mention de la délivrance du brevet: **08.04.2020**
(21) Numéro de dépôt: 13193487.9
(22) Date de dépôt: 19.11.2013
(51) Int. Cl.: A61H 7/00, A61H 15/00, A61N 5/06, A61H 15/02, A61N 1/30, A61H 23/02

(54) **Appareil de massage avec tête de massage équipee de rouleaux de massage**
Massagegerät mit einem Massagekopf, der mit Massagerollen ausgestattet ist
Massage apparatus with massage head provided with a massage roller

(30) Priorité: 22.11.2012 FR 1261108
(43) Date de publication de la demande: 28.05.2014
(73) Titulaire: SEB S.A., 69130 Ecully (FR)
(72) Inventeur: Giraud, Camille, 69001 LYON (FR); Mandica, Franck, 69340 FRANCHEVILLE (FR)
(74) Mandataire: Guéry-Jacques, Géraldine

(56) Documents cités:
- FR-A- 934 070
- FR-A- 1 017 694
- FR-A1- 2 809 952
- FR-A1- 2 902 318
- US-A1- 2008 306 415

## Description

La présente invention concerne le domaine des appareils de traitement de la peau, notamment celle du visage. L'appareil selon l'invention permet, pour le moins, le massage de la peau afin de lui donner de la tonicité. L'appareil de massage selon l'invention trouvera son application chez personnes désireuses de soigner leur esthétique en remodelant, raffermissant et rajeunissant leur peau, notamment celle du visage.

Les appareils de massage de la peau se composent généralement d'un corps muni de moyens de motorisation et d'une tête de massage qui comprend des éléments de massage configurés pour être activés sous l'action des moyens de motorisation, par le biais d'un mécanisme de transmission. Parmi l'art antérieur existant dans ce domaine, il est connu les brevets ou demandes de brevet EP 0 666 071 A1, FR 2 589 726 A1, EP 1 973 510 B1.

Dans le document EP 0 666 071 A1, l'appareil de massage comprend deux éléments de la forme de pale qui tournent en sens inversés pour rapprocher lesdites pales qui plissent la peau. Dans une variante, cet appareil de massage comprend un élément filaire souple qui forme une boucle et dont les deux extrémités sont entraînées en rotation en sens inversés, permettant à la partie en boucle de plisser la peau.

Dans le document FR 2 589 726 A1, l'appareil de massage comprend deux rouleaux agencés au niveau d'une surface d'application, selon deux axes parallèles entre eux et écartés l'un par rapport à l'autre. Un mécanisme de transmission permet d'entraîner les deux rouleaux dans un même sens de rotation. Des moyens de modification de l'écartement des rouleaux sont agencés sur l'appareil et des moyens de rappel permettent d'écarter les rouleaux. Lors de l'utilisation de l'appareil de massage, les rouleaux en position initiale écartée, sont entrainés dans le même sens de rotation, l'un des rouleaux cherchant à se rapprocher de l'autre des rouleaux grâce aux moyens de modification de l'écartement et aux efforts exercés sur ledit rouleau en contact sur la peau, tandis que les moyens de rappel tendent à ramener ledit rouleau en position écartée par rapport à l'autre.

Dans le document EP 1 973 510 B1, l'appareil de massage comprend deux rouleaux agencés au niveau d'une surface d'application et disposés selon des axes parallèles entre eux, les rouleaux étant écartés l'un de l'autre. L'appareil de massage comprend des mécanismes de commande des rouleaux agencés autoriser la rotation du premier rouleau dans un sens et bloquer la rotation du premier rouleau dans l'autre sens et, inversement pour le second rouleau. Ainsi, lorsque l'appareil de massage est appliqué sur la peau et déplacé dans un sens, seul le premier rouleau tourne dans un premier sens de rotation, ce qui permet de plisser la peau et, inversement, lorsque ledit appareil de massage est déplacé dans l'autre sens, seul le second rouleau tourne dans un second sens de rotation, ce qui permet aussi de plisser la peau.

Le document FR934070 décrit un appareil de massage comprenant deux rouleaux entrainés en rotation dont un premier rouleau est fixe sur son axe de rotation et un deuxième rouleau est réglable manuellement pour s'approcher ou s'éloigner du premier rouleau.

Le document FR1017694 décrit un appareil de massage comprenant deux rouleaux entrainés en rotation, les deux rouleaux étant soumis à une action de rappel pour plaquer l'un contre l'autre en position initiale.

Le document FR2902318 décrit un appareil de massage à aspiration comprenant deux rouleaux entrainés en rotation, lesdits rouleaux étant écartés par des ressorts. Lorsque les rouleaux sont soumis à un effort d'aspiration, ils se rapprochent à l'encontre de l'action de rappel des ressorts.

Le document FR2809952 décrit un appareil de massage à aspiration comprenant deux rouleaux se rapprochant l'un par rapport à l'autre par aspiration contre une contraine élastique d'un ressort.

L'objet de l'invention est de concevoir un appareil de massage permettant d'optimiser le travail de la peau par plissage de celle-ci en vue de travailler la peau et les muscles du visage de façon plus complète, agréable, efficace, par rapport aux appareils de massage de l'art antérieur. Tout en évitant les sensations désagréables lors de l'application de l'appareil de massage sur la peau.

A cet effet, l'invention concerne un appareil de massage comprenant un corps qui comporte des moyens de motorisation et une tête de massage. Des moyens d'assemblage sont agencés entre le corps et la tête de massage. La tête comprend une surface d'application qui est positionnée parallèlement à la surface de peau de l'utilisateur durant son utilisation et, deux rouleaux de massage qui sont agencés selon deux premiers axes longitudinaux parallèles, avec un écartement entre eux, et dépassant partiellement à l'extérieur de la surface d'application. Selon l'invention, la tête de massage comprend un mécanisme de transmission configuré pour être entraîné par les moyens de motorisation et pour entraîner en rotation de manière synchronisée et inversée, les deux rouleaux de sorte que, dans une vue en plan perpendiculaire aux deux axes avec les rouleaux disposés vers le bas, la partie du rouleau située à gauche qui dépasse de la surface d'application, tourne dans le sens trigonométrique et, la partie du rouleau située à droite qui dépasse de la surface d'application, tourne dans le sens horaire. En outre, selon l'invention, la tête de massage comprend un système d'ajustement de l'écartement des rouleaux sous l'action des efforts exercés sur ces rouleaux durant leur rotation, configuré pour définir initialement un écartement minimal entre les rouleaux puis pour ajuster l'écartement des rouleaux jusqu'à atteindre un équilibre des efforts exercés directement et/ou indirectement sur ces rouleaux. Ainsi, les caractéristiques de l'appareil de massage selon l'invention permettent de définir une position initiale des rouleaux avec un écartement minimal entre eux, ce qui, en combinaison avec leur sens de rotation inversés et tels que définis ci-dessus, favorise le plissage immédiat et tonique de la peau dès l'application de l'appareil de massage, tandis que la configuration du système d'ajustement permet ensuite aux rouleaux de s'écarter sous l'action des efforts exercés sur la peau jusqu'à atteindre une position d'équilibre. Cela permet de contrôler et de limiter les efforts de pincement sur la peau pour ne pas être douloureux, en s'adaptant à l'épaisseur de peau, variable selon les zones et les personnes, et à la sensibilité de l'utilisateur.

Les moyens d'assemblage entre la tête de massage et le corps pourront être de diverses conceptions, allant d'une tête de massage réalisée en une seule pièce avec le corps, jusqu'au système d'assemblage amovible entre la tête de massage et le corps.

Dans un mode préférentiel de réalisation de l'appareil de massage objet de l'invention, le mécanisme de transmission comprend deux premières roues dentées identiques qui engrènent entre elles et sont disposées selon des seconds axes, lesdits seconds axes étant parallèles entre eux et aux deux premiers axes. Une desdites premières roues dentées est entrainée en rotation par les moyens de motorisation. En outre, ce mécanisme de transmission comprend deux secondes roues dentées identiques qui engrènent respectivement avec les deux premières roues dentées, lesdites secondes roues dentées étant disposées respectivement selon les premiers axes et assujetties aux rouleaux.

Bien entendu des variantes de réalisation du mécanisme de transmission sont envisageables. On pourrait envisager de remplacer les engrenages par des roues ou poulies et des courroies agencées pour obtenir les sens de rotation recherchés des rouleaux.

Selon l'appareil de massage objet de l'invention, le système d'ajustement comprend des moyens de support des rouleaux configurés pour permettre le rapprochement ou, inversement, l'écartement des rouleaux sous l'action d'efforts exercés dans un sens ou, inversement, dans l'autre sens sur lesdits moyens de support.

Dans un mode de réalisation préférentiel de l'appareil de massage objet de l'invention, les moyens de support des rouleaux comprennent deux potences sur lesquelles sont respectivement montés en liaison pivot selon les premiers axes, les rouleaux. En outre, les potences sont respectivement montées en liaison pivot sur deux arbres agencés respectivement selon les deux seconds axes.

Selon l'appareil de massage objet de l'invention, le système d'ajustement comprend des moyens de rappel configurés pour exercer des efforts sur les rouleaux visant à les rapprocher en deçà d'un seuil de résistance de la peau, lors de l'application de l'appareil de massage. En effet, au-delà de ce seuil, la force des ressorts est inférieure à l'effort de résistance de la peau et donc les rouleaux s'écartent.

Dans un mode préférentiel de réalisation de l'appareil de massage, ces moyens de rappel comprennent des éléments ressorts agencés entre les deux potences ou contre les parois de la tête de massage pour favoriser le rapprochement des deux rouleaux.

Selon l'appareil de massage objet de l'invention, celui-ci comprend des butées configurées pour limiter l'écartement minimal entre les deux rouleaux lors de leur rapprochement sous l'action de moyens de rappel. Cela limite les efforts sur la peau lors de l'application initiale de l'appareil de massage.

Selon l'appareil de massage objet de l'invention, celui-ci comprend des moyens de réglage de la position des butées, configurés pour modifier l'écartement minimal entre les rouleaux. Cela permet d'adapter au mieux l'appareil de massage en fonction de l'utilisateur afin d'empêcher que le plissage de peau lors de l'application initiale de l'appareil de massage soit trop intense.

Selon l'appareil de massage objet de l'invention, les rouleaux de massage sont dans une matière et de forme configurées pour accrocher et travailler la peau sans douleur.

Selon l'appareil de massage objet de l'invention, celui-ci comprend un système d'émission d'ondes au niveau de la surface d'application. Ces ondes peuvent être des ondes électromagnétiques, particulièrement lumineuses, visibles ou dans le domaine de l'infrarouge, ou peuvent être sonores par exemple des ultrasons. Cela permet également de travailler de manière complémentaire, la peau de l'utilisateur. Dans un exemple envisagé de réalisation, ces ondes sont lumineuses de couleur rouge ou orange. Selon l'appareil de massage objet de l'invention, celui-ci comprend un système de distribution d'un produit cosmétique configuré pour distribuer ledit produit cosmétique sur les rouleaux de massage et/ou directement sur la peau d'un utilisateur. Selon la configuration du système de distribution, la distribution du produit cosmétique pourra être réalisée de manière naturelle, manuelle et/ou automatique. Cela permet de traiter la peau au moyen de produits cosmétiques, de manière complémentaire aux rouleaux.

Selon l'appareil de massage objet de l'invention, celui-ci comprend un système vibratoire configuré pour faire vibrer la tête de massage ou seulement les rouleaux. Cela permet de solliciter encore davantage la peau et de la détendre (sensation de bien-être) en effectuant de légères pressions sur la peau en complément du plissage réalisé par les rouleaux.

Selon l'appareil de massage objet de l'invention, les moyens d'assemblage entre le corps et la tête de massage sont configurés pour connecter de manière amovible la tête de massage sur le corps. Cela permet de retirer facilement la tête de massage pour la remplacer par une autre identique ou différente.

Selon l'appareil de massage objet de l'invention, celui-ci comprend un dispositif de traitement ionophorèse transcutanée qui est configuré pour transmettre sur la peau lors de l'application dudit appareil de massage, permettant d'augmenter et/ou d'accélerer la pénétration d'un produit cosmétique qui peut être soit distribué par le système de distribution de produit cosmétique décrit ci-dessus, soit appliqué directement sur la peau par l'utilisateur. L'ionophorèse est un traitement qui a été développée initialement pour l'application dans la peau de médicaments, en particulier pour de la médecine sportive, mais est également envisagée maintenant pour une meilleure pénétration d'un cosmétique. Dans un mode de réalisation, ce dispositif de traitement ionophorèse transcutanée comprend deux électrodes à potentiel électrique différent qui peuvent être agencées sur les rouleaux de massage et/ou sur la surface d'application de la tête de massage. Les réglages de l'appareil de massage selon l'invention, en particulier lorsque celui-ci comprend également un système de distribution de produits cosmétique, permettront de réaliser le traitement par ionophorèse transcutanée avant et /ou pendant l'application du produit cosmétique.

La description suivante met en évidence les caractéristiques et avantages de l'appareil de massage selon l'invention, laquelle s'appuie sur des figures parmi lesquelles :
- La figure 1 est une vue en perspective d'un mode de réalisation de l'appareil de massage selon l'invention ;
- Les figures 2 et 3 illustrent deux positions différentes des rouleaux de massage ;
- La figure 4 montre l'appareil de massage de la figure 1 en vue de dessus et en coupe ;
- La figure 5 illustre le mécanisme de transmission de la figure 1 ;
- Les figures 6 et 7 schématisent un système de distribution de produit cosmétique sur l'appareil de massage ;
- La figure 8 schématise un système vibratoire sur l'appareil de massage ;
- La figure 9 schématise des moyens d'assemblage amovibles entre la tête de massage et le corps ;
- La figure 10 schématise un dispositif de traitement ionophorèse transcutanée sur l'appareil de massage ;
- La figure 11 schématise un système de distribution de produit cosmétique sur l'appareil de massage ;
- La figure 12 schématise un système d'émission d'ondes sur l'appareil de massage ;
- La figure 13 illustre une variante de réalisation d'un mécanisme de transmission sur une tête de massage ;
- Les figures 14A - 14C illustrent des variantes de formes des rouleaux.

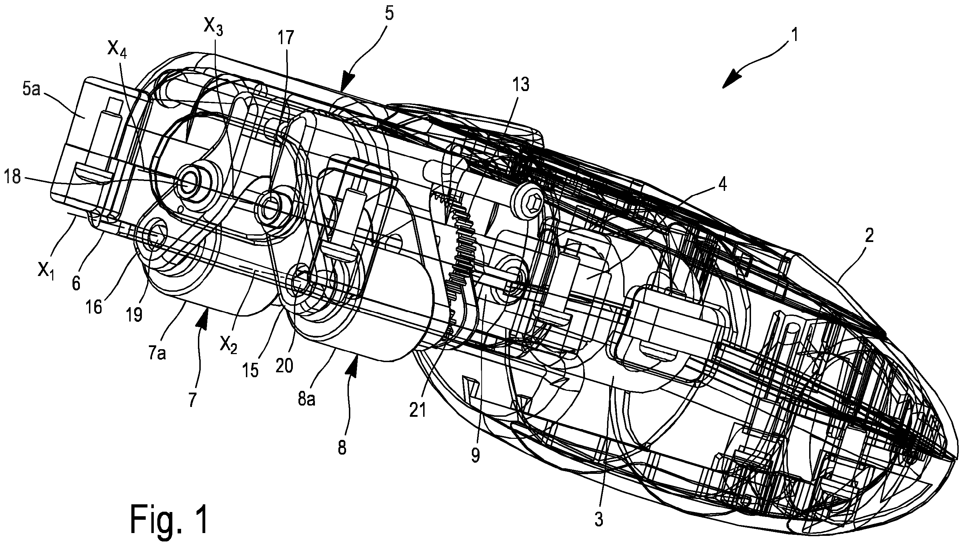

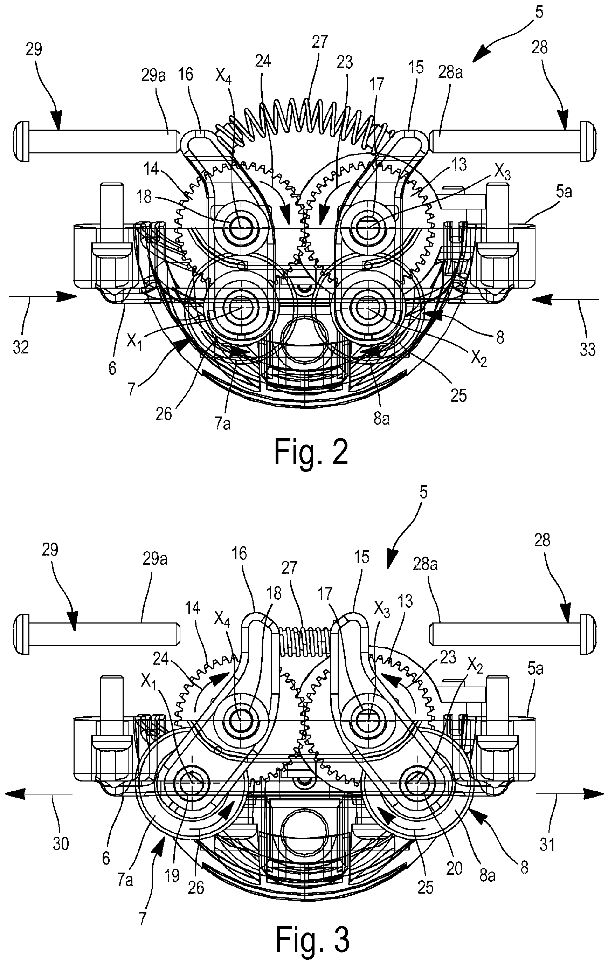

Tel qu'illustré sur les figures 1 et 2, l'appareil de massage 1 comprend un corps 2 à l'intérieur duquel est agencé un moteur 3 alimenté électriquement par raccordement soit à une source électrique externe, au moyen d'une prise électrique et d'un transformateur basse tension, soit à source électrique interne du type batterie rechargeable ou piles jetables. L'appareil de massage 1 comprend également un motoréducteur 4 agencé en sortie du moteur 3 pour réduire sa vitesse de rotation et l'adapter au besoin d'utilisation. L'appareil de massage 1 comprend également une tête de massage 5. Le corps 2 et la tête de massage 5 sont réalisés dans une même pièce moulée. On peut toutefois envisager deux pièces distinctes assemblées entre elles de manière amovible, dans une variante de réalisation.

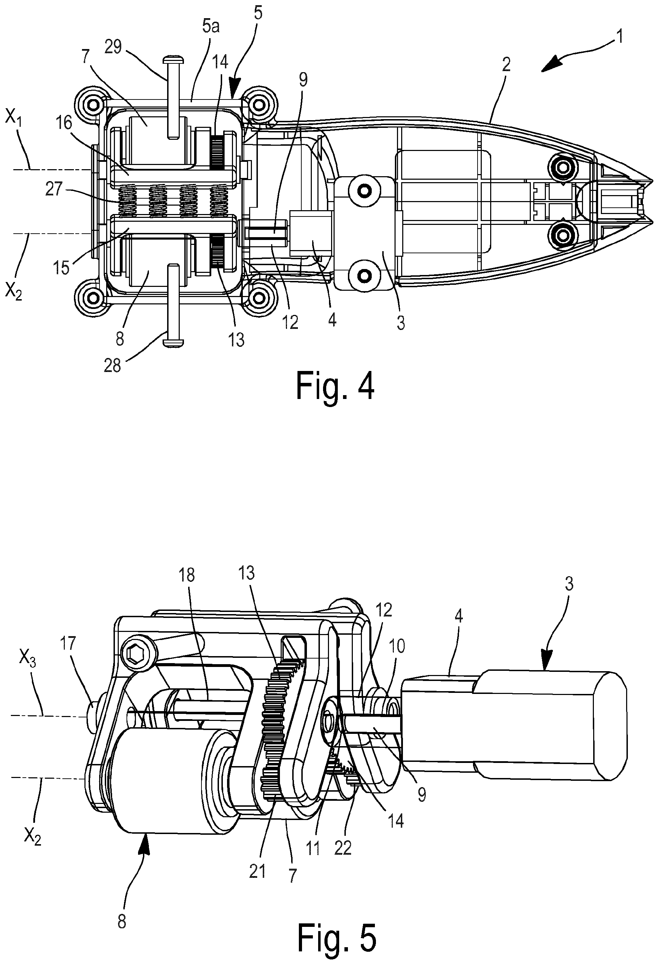

Tel qu'illustré sur les figures 1 à 5 la tête de massage comprend une surface d'application 6 et deux rouleaux de massage 7, 8 dont une partie 7a, 8a dépasse de la surface d'application 6. Ces rouleaux 7, 8 sont disposés selon deux axes X₁, X₂ parallèle entre eux. Selon ce mode de réalisation, le moteur 4 comprend un arbre d'entraînement 9 qui comprend un méplat 10 qui s'engage dans un trou 11 agencé sur un arbre 12. Cet arbre 12 est assujetti à une roue dentée 13. Ainsi le moteur 3 entraîne en rotation la roue dentée 13 selon un axe X₃. Cette roue dentée 13 engrène avec une autre roue dentée 14 de conception identique et disposée selon un axe X₄.

Tel qu'illustré sur les figures 1 à 5, la tête de massage 1 comprend deux potences 15, 16. Ces potences 15, 16 sont montées en liaison pivot d'axes respectifs X₃, X₄ au moyen de deux arbres 17, 18. Ces potences reçoivent en liaison pivot d'axe X1, X2 les rouleaux 7, 8 au moyen de deux arbres 19, 20 montés en liaison pivot sur lesdites potences 15, 16.

Tel qu'illustré sur les figures 1 et 3, la tête de massage comprend deux autres roues dentées 21, 22 de conception identique. La roue dentée 21 est assujettie au rouleau 8 par le biais de l'arbre 20 par exemple au moyen de cannelures (non illustrées) agencées entre ces éléments. De même, la roue dentée 22 est assujettie au rouleau 7 par le biais de l'arbre 19. La roue dentée 21 engrène avec la roue dentée 13. De même, la roue dentée 22 engrène avec la roue dentée 14. Ainsi, lors de l'entraînement de la roue dentée 13 par le biais du moteur 3, qui tourne dans le sens de la flèche 23, la roue dentée 14 tourne dans le sens de la flèche 24, ce qui permet à la roue dentée 21 de tourner dans le sens de la flèche 25 et à la roue dentée 22 de tourner dans le sens de la flèche 26. Ainsi, le rouleau 7 est entraîné dans le sens de la flèche 26 et le rouleau 8 dans le sens de la flèche 25.

Tel qu'illustré sur les figures 2 à 4, des ressorts 27 sont agencés entre les deux potences 15, 16. En position initiale, ces ressorts 27 pour contre les potences 15, 16 et les maintiennent en appui contre les extrémités 28a, 29a de deux vis de réglage 28, 29 agencées sur la tête de massage 5. Ainsi les rouleaux 7 ou 8 sont écartés l'un de l'autre avec un écartement minimal en position initiale. En outre, le réglage des vis 28, 29 permet ainsi de modifier l'écartement minimal entre ces deux rouleaux 7, 8.

Lorsque les parties 7a, 8a des rouleaux 7, 8 sont appliquées sur la peau, les sens des rotations inversés des rouleaux 7, 8 dans le sens des flèches 26, 27 permet de former un pli sur la peau. Les efforts exercés par la peau sur ces rouleaux, lorsque ces efforts sont supérieurs aux efforts exercés par les ressorts 27, permettent alors de comprimer ces ressorts 27 et d'écarter les rouleaux dans le sens des flèches 30, 31 illustrées en figure 3. Au contraire, si ces efforts exercés par la peau sur les rouleaux 7, 8 sont inférieurs aux efforts exercés par les ressorts 27, ces ressorts 27 se détendent et ramènent les rouleaux 7, 8 dans le sens des flèches 32, 33 illustrées en figure 2, jusqu'à atteindre une position d'équilibre.

Des variantes de réalisation de mécanismes de transmission sur la tête de massage 5, fonctionnant selon le même principe, sont envisageables sans sortir du cadre de l'invention. On peut notamment agencer les ressorts entre les potences 15, 16 et le carter 5a de la tête de massage 5 dans lequel est agencé le mécanisme de transmission. Ces ressorts travailleront en extension pour ramener les rouleaux 7, 8 dans une position initiale avec un écartement minimal.

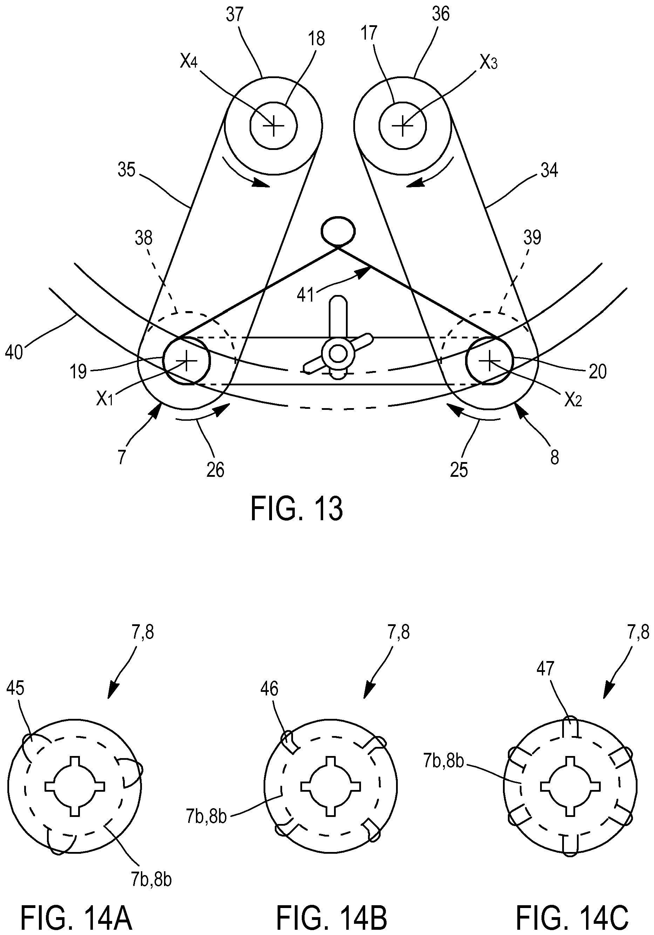

Tel qu'illustré en figure 13, on peut également prévoir un entraînement des rouleaux 7, 8 au moyens de courroies 34, 35 et poulies 36, 37, 38, 39 entraînées par le biais du moteur 3 en remplacement des roues dentées 13, 14, 21, 22 engrenant entre elles. De même les potences 15, 16 peuvent être remplacées par un système de guidage 40 des arbres 19, 20 assujettis aux rouleaux 7, 8. Un ressort de rappel 41 est en outre agencé entre les arbres 19, 20 pour ramener les rouleaux vers un écartement minimal.

D'autres caractéristiques sont envisageables sur l'appareil de massage 1 objet de l'invention.

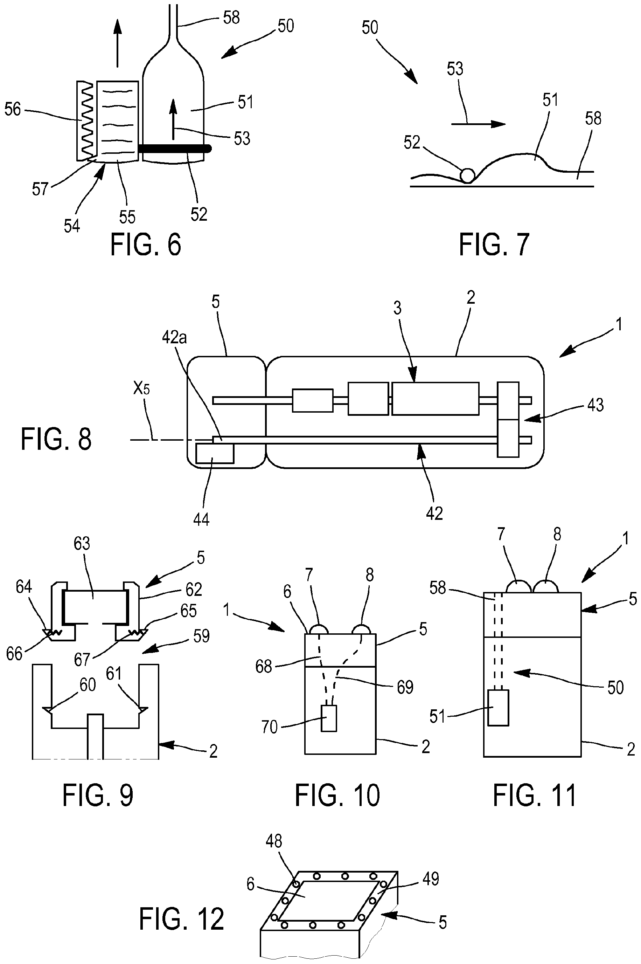

Tel qu'illustré en figure 8, l'appareil de massage 1 comprend un arbre 42 qui est entraîné en rotation selon un axe X₅ par le moteur 3, au moyen d'un système d'engrenage 43 connu de l'homme du métier. L'extrémité 42a de de cet arbre 42 est agencée dans la tête de massage 5, par exemple sur le carter 5a ou les potences 15, 16, et reçoit une masselotte 44 qui tourne en balourd autour de l'axe X₅ et permet de faire vibrer la tête de massage 5 durant l'application de l'appareil de massage 1. D'autres variantes de système vibratoire restent envisageables. On peut notamment envisager de faire vibrer uniquement les rouleaux 7, 8.

Tel qu'illustré sur les figures 14A - 14C, la partie externe 7b, 8b des rouleaux de massage 7, 8 peut présenter des formes variables comme, par exemple, une forme de bourrelet 45 en figure 14A, une forme de crochet 46 en figure 14B et une forme de picot 47 en figure 14C. D'autres formes restent envisageables. En outre, les rouleaux de massage 7, 8 sont dans une matière telle que de l'élastomère permettant, en combinaison avec ces formes, de mieux accrocher la peau lors de l'application de l'appareil de massage 1.

Dans une variante illustrée en figure 12, l'appareil de massage 1 comprend au niveau de la surface d'application 6 sur la tête de massage 5, des diodes luminescentes 48 qui sont commandées par un boîtier électronique (non illustré) agencé à l'intérieur du corps 2. Ces diodes luminescentes 48 pourront être allumées soit automatiquement lors de l'actionnement de la tête de massage 5, soit séparément au moyen du bouton de commande distinct (non illustré). Les diodes luminescentes 48 seront disposées par exemple sur le bord périphérique 49 de la surface d'application 186, voire réparties sur ladite surface d'application 6 en dehors des trajectoires des rouleaux de massage 7, 8. On pourra utiliser des diodes luminescentes de différentes couleur selon la longueur d'onde souhaitée et/ou le traitement recherché, voire prévoir un boîtier de commande permettant de modifier la longueur d'onde de ces diodes luminescentes 48.

Dans une variante illustrée en figures 6, 7 et 11, on prévoit sur l'appareil de massage 1 un système de distribution de produit cosmétique 50 qui comprend un réservoir de produit 51 souple sur lequel appuie une barrette 52 qui se déplace en translation selon la flèche 53 le long du réservoir de produit 205. Un système d'actionnement 54 permet de déplacer la barrette 52 selon la flèche 53. Ce système d'actionnement 54 est par exemple constitué d'un bouton 55 solidaire de la barrette 52 et translatant dans le sens de cette flèche 53 lors d'une action manuelle. Une pièce crantée 56 permet de maintenir un cran 57 sur le bouton 55 lors de chaque impulsion sur celui-ci pour maintenir en position la barrette 52 au fur et à mesure de son avancement sur le réservoir de produit 51. Le système de distribution 50 comprend un tuyau 58 dont l'agencement permet de distribuer le produit cosmétique au niveau de la surface d'application 6, soit entre les rouleaux 7,8 soit au travers des rouleaux 7, 8, comme illustré en figure 11. D'autres variantes de système de distribution de produit cosmétique sont envisageables. On peut notamment prévoir un système d'actionnement automatique permettant de distribuer de manière continue et régulière, par exemple au moyen d'une pompe, le produit cosmétique. On peut également prévoir un système de distribution de produit cosmétique de manière naturelle, le produit cosmétique étant diffusé régulièrement grâce à des phénomènes physiques ne nécessitant pas un actionnement extérieur.

Tel qu'illustré au regard de la figure 9, on peut envisager des variantes selon lesquelles la tête de massage 5 peut être montée et démontée rapidement du corps 2. Cela permet de changer facilement de tête de massage 5. A ce titre, on peut envisager un système de fixation 59 amovible entre la tête de massage 5 et le corps 2. Par exemple, comme illustré sur la figure 12, le corps 2 peut comprendre des encoches 60, 61 et la tête de massage 5 comprend une pièce 62 qui reçoit le mécanisme de transmission 63 du mouvement des rouleaux de massage 7, 8 connecté à l'arbre 9 du moteur 3. Cette pièce 62 comprend des crans 64, 65, des moyens de rétractation 66, 67 des crans 219, 220 permettant de les escamoter pour leur positionnement dans les encoches 60, 61 et aussi leur retrait de ces encoches 60, 61.

Dans une variante de réalisation illustrée en figure 10, l'appareil de massage 1 comprend deux électrodes 68, 69 qui présentent un potentiel électrique différent. Ces électrodes 68, 69 peuvent être agencées sur les rouleaux de massage 7, 8 ou être agencées sur la surface d'application 6. Ces électrodes 68, 69 sont alimentées par une source électrique 70 agencé dans le corps 2. Cette conception permet de réaliser un traitement par inophorèse transcutanée avant ou pendant l'application d'un produit cosmétique sur la peau, ce qui permet d'accélérer la pénétration du produit cosmétique. Ce produit cosmétique peut être distribué de manière naturelle, manuelle ou automatique par l'appareil de massage 1, voire appliqué directement sur la peau par l'utilisateur.

Ces caractéristiques complémentaires précitées des différentes variantes de réalisation de l'appareil de massage 1 pourront éventuellement être mise en œuvre en combinaison avec l'un ou l'autre des mécanismes de transmission sur la tête de massage tels que décrits ci-dessus, voire avec d'autres variantes envisageables dans le cadre de l'invention.

## Revendications

1. Appareil de massage (1) du visage comprenant un corps (2) qui comporte un moteur (3) alimenté électriquement et une tête de massage (5), des moyens d'assemblage (59) étant agencés entre le corps et la tête de massage, ladite tête comprenant une surface d'application (6) et deux rouleaux de massage (7, 8) agencés selon deux premiers axes (X₁, X₂) longitudinaux parallèles, avec un écartement entre eux, et dépassant partiellement à l'extérieur de la surface d'application, la tête de massage comprenant, d'une part, un mécanisme de transmission configuré pour être entraîné par le moteur (3) et pour entraîner en rotation de manière synchronisée et inversée, les deux rouleaux de sorte que, dans une vue en plan perpendiculaire aux deux axes avec les deux rouleaux disposés en bas, la partie (7a) du rouleau (7) située à gauche qui dépasse de la surface d'application, tourne dans le sens trigonométrique et, la partie (8a) du rouleau (8) située à droite qui dépasse de la surface d'application, tourne dans le sens horaire et, d'autre part, un système d'ajustement de l'écartement des rouleaux, **caractérisé en ce que** le système d'ajustement de l'écartement des rouleaux est réalisé sous l'action des efforts exercés sur ces rouleaux durant leur rotation, configuré pour définir initialement un écartement minimal entre les rouleaux puis pour ajuster l'écartement jusqu'à atteindre un équilibre des efforts exercés sur ces rouleaux.

2. Appareil de massage (1) selon la revendication 1, dans lequel le mécanisme de transmission comprend :
- deux premières roues dentées (13, 14) identiques qui engrènent entre elles et sont disposées selon des seconds axes (X₃, X₄), lesdits seconds axes étant parallèles entre eux et aux deux premiers axes (X₁, X₂), une desdites premières roues dentées (13) étant entrainée en rotation par le moteur (3) et,
- deux secondes roues dentées (21, 22) identiques qui engrènent respectivement avec les deux premières roues dentées, lesdites secondes roues dentées étant disposées respectivement selon les premiers axes et assujetties aux rouleaux (7, 8).

3. Appareil de massage (1) selon la revendication 1 ou 2, dans lequel le système d'ajustement comprend des moyens de support (15, 16) des rouleaux (7, 8) configurés pour permettre le rapprochement ou, inversement, l'écartement des rouleaux sous l'action d'efforts exercés dans un sens ou, inversement, dans l'autre sens sur lesdits moyens de support.

4. Appareil de massage (1) selon la revendication 3 rattachée à la revendication 2, dans lequel les moyens de support des rouleaux (7, 8) comprennent deux potences (15, 16) sur lesquelles sont respectivement montés en liaison pivot selon les premiers axes (X₁, X₂), les rouleaux, lesdites potences étant respectivement montées en liaison pivot sur deux arbres (17, 18) agencés respectivement selon les deux seconds axes (X₃, X₄).

5. Appareil de massage (1) selon l'une des revendications 1 à 4, dans lequel le système d'ajustement comprend des moyens de rappel (27, 41) configurés pour exercer des efforts sur les rouleaux (7, 8) visant à les rapprocher en deçà d'un seuil de résistance.

6. Appareil de massage (1) selon la revendication 5 rattachée à la revendication 4, dans lequel les moyens de rappel comprennent des éléments ressorts (27, 41) configurés pour favoriser le rapprochement des deux rouleaux (7, 8).

7. Appareil de massage (1) selon l'une des revendications 5 ou 6, lequel comprend des butées (28a, 29a) configurées pour limiter l'écartement minimal entre les deux rouleaux (7, 8) lors de leur rapprochement sous l'action des moyens de rappel.

8. Appareil de massage (1) selon la revendication 7, lequel comprend des moyens de réglage (28, 29) de la position des butées, configurés pour modifier l'écartement minimal entre les rouleaux (7, 8).

9. Appareil de massage (1) selon l'une des revendications 1 à 8, dans lequel les rouleaux de massage (7, 8) sont dans une matière telle que l'élastomère et de forme (45, 46, 47) telle qu'une forme de bourrelet/crochet/picot configurées pour accrocher et travailler la peau sans douleur.

10. Appareil de massage (1) selon l'une des revendications 1 à 9, lequel comprend un système d'émission (48) d'ondes au niveau de la surface d'application (6).

11. Appareil de massage (1) selon l'une des revendications 1 à 10, lequel comprend un système de distribution (50) d'un produit cosmétique configuré pour distribuer ledit produit cosmétique sur les rouleaux de massage (7, 8) et/ou directement sur la peau d'un utilisateur.

12. Appareil de massage (1) selon l'une des revendications 1 à 11, lequel comprend un système vibratoire (42, 44) configuré pour faire vibrer la tête de massage (5) ou les rouleaux.

13. Appareil de massage (1) selon l'une des revendications 1 à 12, dans lequel les moyens d'assemblage (59) entre le corps (2) et la tête de massage (5) sont configurés pour connecter de manière amovible la tête de massage sur le corps.

14. Appareil de massage (1) selon l'une des revendications 1 à 13, lequel comprend un dispositif de traitement ionophorèse transcutanée (68, 69, 70) qui est configuré pour transmettre sur la peau lors de l'application dudit appareil de massage, permettant d'augmenter et/ou d'accélérer la pénétration d'un produit cosmétique.

## Patentansprüche

1. Massagegerät (1) für das Gesicht, umfassend einen Körper (2), der einen Motor (3) beinhaltet, der elektrisch versorgt wird, und einen Massagekopf (5), wobei Montagemittel (59) zwischen dem Körper und dem Massagekopf angeordnet sind, wobei der Kopf eine Anlegeoberfläche (6) und zwei Massagerollen (7, 8) umfasst, die entlang zweier erster paralleler Längsachsen (X₁, X₂) in einem Abstand zwischen ihnen angeordnet sind, und teilweise nach außerhalb der Anlegeoberfläche überstehen,
wobei der Massagekopf umfasst,
einerseits einen Übertragungsmechanismus, der konfiguriert ist, um von dem Motor (3) angetrieben zu werden, und um auf synchronisierte und umgekehrte Art die beiden Rollen in Drehung anzutreiben, sodass sich in einer Ebene senkrecht zu den beiden Achsen mit den beiden unten angeordneten Rollen gesehen, der links befindliche Teil (7a) der Rolle (7), die über die Anlegeoberfläche übersteht, in die trigonometrische Richtung dreht und sich der rechts befindliche Teil (8a) der Rolle (8), die über die Anlegeoberfläche übersteht, im Uhrzeigersinn dreht, und
andererseits ein System zum Anpassen des Abstandes der Rollen,
**dadurch gekennzeichnet, dass** das System zum Anpassen des Abstandes der Rollen unter der Wirkung der Kräfte realisiert wird, die während deren Drehung auf diese Rollen ausgeübt werden, konfiguriert, um ursprünglich einen minimalen Abstand zwischen den Rollen zu definieren, um danach den Abstand anzupassen, um ein Gleichgewicht der Kräfte zu erreichen, die auf diese Rollen ausgeübt werden.

2. Massagegerät (1) nach Anspruch 1, wobei der Übertragungsmechanismus Folgendes umfasst:
- zwei erste identische Zahnräder (13, 14), die ineinander eingreifen und die entlang von zweiten Achsen (X₃, X₄) angeordnet sind, wobei die zweiten Achsen zueinander und zu den beiden ersten Achsen (X₁, X₂) parallel sind, wobei eines der ersten Zahnräder (13) durch den Motor (3) in Drehung angetrieben wird, und
- zwei zweite identische Zahnräder (21, 22), die jeweils in die zwei zwei ersten Zahnräder eingreifen, wobei die zweiten Zahnräder jeweils entlang der ersten Achsen angeordnet sind, und den Rollen (7, 8) unterworfen sind.

3. Massagegerät (1) nach Anspruch 1 oder 2, wobei das System zum Anpassen Trägermittel (15, 16) für die Rollen (7, 8) umfasst, die konfiguriert sind, um die Annäherung oder umgekehrt die Entfernung der Rollen unter der Wirkung der Kräfte, die in einer Richtung oder umgekehrt in der anderen Richtung auf die Trägermittel ausgeübt werden, zu erlauben.

4. Massagegerät (1) nach Anspruch 3, angegliedert an den Anspruch 2, wobei die Trägermittel für die Rollen (7, 8) zwei Stützen (15, 16) umfassen, auf denen jeweils entlang der ersten Achsen (X₁, X₂) die Rollen in Schwenkverbindung montiert sind, wobei die Stützen jeweils in Schwenkverbindung auf zwei Wellen (17, 18) montiert sind, die jeweils entlang der beiden zweiten Achsen (X₃, X₄) angeordnet sind.

5. Massagegerät (1) nach einem der Ansprüche 1 bis 4, wobei das System zum Anpassen Rückstellmittel (27, 41) umfasst, die konfiguriert sind, um Kräfte auf die Rollen (7, 8) auszuüben, die darauf abzielen, sie diesseits einer Widerstandsschwelle anzunähern.

6. Massagegerät (1) nach Anspruch 5, angegliedert an den Anspruch 4, wobei die Rückstellmittel Federelemente (27, 41) umfassen, die konfiguriert sind, um die Annäherung der beiden Rollen (7, 8) zu unterstützen.

7. Massagegerät (1) nach einem der Ansprüche 5 oder 6, das Anschläge (28a, 29a) umfasst, die konfiguriert sind, um den minimalen Abstand zwischen den beiden Rollen (7, 8) bei deren Annäherung unter der Wirkung der Rückstellmittel zu bregrenzen.

8. Massagegerät (1) nach Anspruch 7, das Mittel zum Einstellen (28, 29) der Position der Anschläge umfasst, die konfiguriert sind, um den minimalen Abstand zwischen den Rollen (7, 8) zu ändern.

9. Massagegerät (1) nach einem der Ansprüche 1 bis 8, wobei die Massagerollen (7, 8) aus einem Material wie Elastomer sind, und in einer Form (45, 46, 47) wie einer Form eines Wulsts/Hakens/Spitzkeils, konfiguriert, um die Haut schmerzfrei zu erfassen und zu bearbeiten.

10. Massagegerät (1) nach einem der Ansprüche 1 bis 9, das ein Wellenemissionssystem (48) im Bereich der Anlegeoberfläche (6) umfasst.

11. Massagegerät (1) nach einem der Ansprüche 1 bis 10, das ein System zum Verteilen (50) eines Kosmetikprodukts umfasst, das konfiguriert ist, um das Kosmetikprodukt auf den Massagerollen (7, 8) und/oder direkt auf der Haut eines Nutzers zu verteilen.

12. Massagegerät (1) nach einem der Ansprüche 1 bis 11, das ein Schwingsystem (42, 44) umfasst, das konfiguriert ist, um den Massagekopf (5) oder die Rollen schwingen zu lassen.

13. Massagegerät (1) nach einem der Ansprüche 1 bis 12, wobei die Montagemittel (59) zwischen dem Körper (2) und dem Massagekopf (5) konfiguriert sind, um den Massagekopf abnehmbar mit dem Körper zu verbinden.

14. Massagegerät (1) nach einem der Ansprüche 1 bis 13, das eine Vorrichtung zur transkutanen lonophoresebehandlung (68, 69, 70) umfasst, die konfiguriert ist, um beim Anlegen des Massagegerätes auf die Haut zu übertragen, wodurch erlaubt wird, das Eindringen eines Kosmetikproduktes zu verstärken und/oder zu beschleunigen.

## Claims

1. Massage device (1) for the face including a body (2) which contains an electrically-powered motor (3) and a massage head (5), with installation mechanism (59) located between the body and the massage head; said head includes an application surface (6) and two massage wheels (7, 8) located along two first parallel longitudinal axes (X₁, X₂), which are separated and extend partially beyond the application surface, where the massage head includes, on the one hand, a transmission mechanism designed to be driven by the motor (3) to cause the synchronized and inverse rotation of the two wheels so that, when seen in a plane perpendicular to two axes with the two wheels at the bottom, section (7a) of wheel (7) located on the left which extends beyond the application surface, turns anti-clockwise and section (8a) of wheel (8) located on the right which extends outside the application surface, turns clockwise and, on the other hand, a wheel spacing adjustment system, **characterised in that** the wheel spacing adjustment system is carried out under the action of forces placed on these wheels when they rotate, designed to initially create a minimum distance between the wheels and then to adjust the distance between them until a position of equilibrium of the forces on these wheels is reached.

2. Massage device (1) in accordance with claim 1 in which the transmission mechanism includes:
- two first identical geared wheels (13, 14) which intermesh and are located along second axes (X₃, X₄) which are parallel to one another and to the two first axes (X₁, X₂), one of said first geared wheels (13) being rotated by the motor (3) and,
- two second identical geared wheels (21, 22) which mesh respectively with the first two geared wheels, said second geared wheels are located respectively along the first axes and driven by wheels (7, 8).

3. Massage device (1) in accordance with claim 1 or 2 in which the adjustment system includes a support mechanism (15, 16) of wheels (7, 8) designed to increase or inversely decrease the wheel spacing based on the action of forces exerted on said support mechanism in one direction or the other.

4. Massage device (1) in accordance with claim 3 attached to claim 2, in which wheel (7, 8) support system includes two supports (15, 16) on which the wheels are respectively mounted using a pivot connection along the first axes (X₁, X₂), the wheels, said supports are mounted respectively as a pivot connection on two shafts (17, 18) located respectively along two second axes (X₃, X₄).

5. Massage device (1) in accordance with one of claims 1 to 4, in which the adjustment system includes recall mechanism (27, 41) designed to exert forces on the wheels (7, 8) to move them together below a resistance threshold.

6. Massage device (1) in accordance with claim 5 attached to claim 4, in which the recall mechanism include spring elements (27, 41) designed to move two wheels (7, 8) closer together.

7. Massage device (1) in accordance with one of claims 5 or 6, which includes stops (28a, 29a) designed to limit the minimum distance between the two wheels (7, 8) when they are moved together by the recall mechanism.

8. Massage device (1) in accordance with claim 7 which includes adjustment mechanism (28, 29) of the position of the stops designed to modify the minimum distance between wheels (7, 8).

9. Massage device (1) in accordance with one of claims 1 to 8, in which massage wheels (7, 8) are in a material such as elastomer and of shape (45, 46, 47) such as a shape of bulge/hook/pin designed to catch and work the skin without pain.

10. Massage device (1) in accordance with one of claims 1 to 9, which includes a wave emission system (48) on the application surface (6).

11. Massage device (1) in accordance with one of claims 1 to 10, which includes a distribution system (50) of a cosmetic product designed to distribute said cosmetic product on the massage wheels (7, 8) and/or directly on the user's skin.

12. Massage device (1) in accordance with one of claims 1 to 11, which includes a vibration system (42, 44) designed to vibrate massage head (5) or the wheels.

13. Massage device (1) in accordance with one of claims 1 to 12, in which installation mechanism (59) between the body (2) and the massage head (5) is designed to connect, in a removable manner, the massage head on the body.

14. Massage device (1) in accordance with one of claims 1 to 13, which includes a transdermal ionization treatment device (68, 69, 70) which is designed to transfer to the skin during the application of said massage device, making it possible to increase and/or accelerate penetration of a cosmetic product.
